# EUROPÄISCHE PATENTANMELDUNG

(11) **EP 0 740 926 A2**
(43) Veröffentlichungstag der Anmeldung: **06.11.1996**
(21) Anmeldenummer: 96105805.4
(22) Anmeldetag: 12.04.1996
(51) Int. Cl.: A61B 17/39

(54) **Elektrochirurgische Vorrichtung zur Erzeugung eines Lichtbogens**

(30) Priorität: 03.05.1995 DE 19516238
(71) Anmelder: Gebr. Berchtold GmbH & Co., 78532 Tuttlingen (DE)
(72) Erfinder: Wurzer, Helmut, 80538 München (DE); Mäckel, Rainer, 53639 Köningswinter (DE); Liess, Hans-Dieter, Dr., 82541 Seeheim/Münsing (DE); Müller, Wolfgang, Dr.-Ing., 78532 Tuttlingen (DE)
(74) Vertreter: Manitz, Gerhart, Dr.

(57) **Zusammenfassung**

Die Erfindung betrifft eine elektrochirurgische Vorrichtung zur berührungslosen Oberflächenkoagulation von Biogewebe mittels einer Hochfrequenz-Schneidelektrode 12. Zur Schaffung einer ionisierten Strecke für die erleichterte Lichtbogenbildung wird in einem rohrförmigen Instrumentenkörper 11 ein Aerosolstrahl 20 gebildet, der aus dem distalen Ende 13 des Instrumentenkörpers 11 austritt.

## Beschreibung

Die Erfindung betrifft ein Verfahren nach dem Oberbegriff des Patentanspruchs 1 und eine elektrochirurgische Vorrichtung nach dem Oberbegriff des Anspruches 7.

Bei stark durchblutetem Biogewebe besteht häufig das Erfordernis, unter Verwendung der Hochfrequenz-Chirurgie Gewebeoberflächen berührungslos und gleichmäßig zu koagulieren. Konventionell wird dies mit der sogenannten Sprüh-Koagulation bzw. Spray-Koagulation bei hohen Hochfrequenzspannungen im Bereich von Kilovolt bewerkstelligt. Ein Nachteil dieser konventionellen Technik ist die Unregelmäßigkeit des Lichtbogenbereichs und die Gefahr, die Gewebeoberfläche zu karbonisieren.

Eine Verbesserung wurde dadurch erzielt, daß durch Einblasen eines Edelgases z. B. von Argon ein Ionisierungspfad geschaffen wurde, der zur Verbesserung der Gleichmäßigkeit bei der Koagulation von Gewebeoberflächen beitrug. Ein derartiges Verfahren ist z. B. in der EP 0 353 177 A1 beschrieben. Nachteil dieses Verfahrens ist die Verwendung des recht teuren und in Kliniken häufig nicht verfügbaren Edelgases.

Das Ziel der vorliegenden Erfindung besteht darin, ein Verfahren und eine Vorrichtung der eingangs genannten Gattung zu schaffen, mittels denen ein Ionisierungspfad im Bereich der aktiven Elektrode mit einfacheren Maßnahmen zur Verfügung gestellt wird.

Zur Lösung dieser Aufgabe sind die Merkmale der Patentansprüche 1 und 7 vorgesehen.

Der Erfindung liegt der Grundgedanke zugrunde, daß ein Ionisierungspfad auch mit anderen Medien als Edelgasen möglich ist. Als vorteilhaft hat sich hierbei die Erzeugung eines Aerosols aus einer niederprozentigen Kochsalzlösung herausgestellt. Die Kombination von Hochfrequenz an der aktiven Elektrode einer elektro-chirurgischen Vorrichtung mit einer NaCl-Lösung ist zwar bekannt, doch wurde diese bisher nur verwendet, um ein Ankleben der Elektroden am Biogewebe zu verhindern.

Erfindungsgemäß wird dem gegenüber ein Aerosol mit einem gewissen Spüldruck von z. B. 3,5 bar um die Elektrode herumgespült bzw. herumgeblasen, wodurch ein Ionisierungskegel mit definierter Dimensionierung entsteht.

Als positive Effekte des erfindungsgemäßen Verfahrens ergeben sich die Verwendung von verfügbaren Medien, z. B. destilliertem Wasser, CO₂, NaCl-Lösung, eine gleichmäßige Koagulation des Biogewebes in einer festumgrenzten Fläche, eine geringe Gefahr einer Karbonisierung, eine Kühlung der Elektrode, keine, sonst übliche Rauchbildung und keinerlei Geruchsbildung durch Verbrennen von Gewebe.

Vorteilhafte Ausführungen des erfindungsgemäßen Verfahrens sind durch die Ansprüche 2 bis 6 und vorteilhafte Ausführungsformen der erfindungsgemäßen Vorrichtung durch die Ansprüche 8 bis 10 gekennzeichnet.

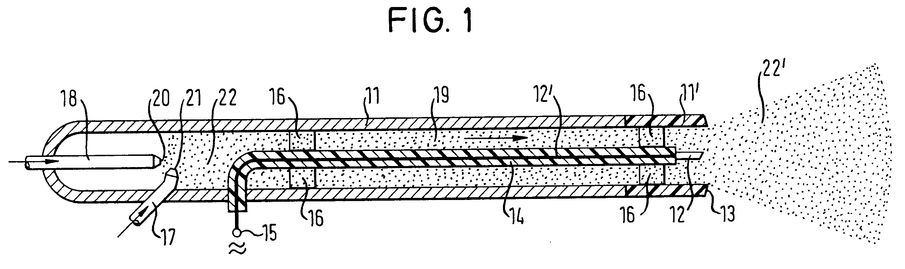

Die Erfindung wird im folgenden beispielsweise anhand der Zeichnung beschrieben; in dieser zeigt
- Figur 1: einen schematischen Schnitt einer rein beispielsweisen Ausführungsform einer erfindungsgemäßen elektrochirurgischen Vorrichtung zur Erzeugung eines Aerosolstrahls um eine hochfrequenz-chirurgische Schneidelektrode herum.

In einem von der Hand des Benutzers ergreifbaren hohlzylinderförmigen Instrumentenkörper 11 ist koaxial eine hochfrequenz-chirurgische Schneidelektrode 12 angeordnet, die gegenüber dem distalen offenen Ende 13 des rohrförmigen Instrumentenkörpers 11 ein Stück zurückversetzt ist und mit einer Zuleitung 12' verbunden ist, die sich - von einer Isolierung 14 umgeben - mit allseitigem Abstand von der Innenwand des rohrförmigen Instrumentenkörpers 11 nach hinten erstreckt, wo im Bereich des proximalen Endes seitlich ein Hochfrequenzanschluß 15 vorgesehen ist, an den eine geeignete Hochfrequenzspannung, die von einem nicht dargestellten Hochfrequenzgenerator erzeugt wird, anlegbar ist. Die zugehörige Neutralelektrode ist an geeigneter Stelle des Patientenkörpers angeordnet. Der distale Bereich des im übrigen aus Metall bestehenden Instrumentenkörpers 11 ist aus einem isolierenden Material 11' hergestellt, welches sich nach hinten bis über die Isolierung 14 der Zuleitung 12' erstreckt.

Die Zuleitung 12' mit der Isolierung 14 wird durch geeignete Halterungen 16 vorzugsweise koaxial innerhalb des Instrumentenkörpers 11 gehalten. Die einzelnen Halterungen 16 sind jeweils über den Umfang um die Isolierung 14 herum mit solchem Abstand angeordnet, daß dazwischen in axialer Richtung ein ausreichend dimensionierter Strömungsdurchgang vorliegt.

Am proximalen Ende des Instrumentenkörpers 11 sind seitlich bzw. axial zwei Zufuhrrohre 17, 18 in das Innere des Instrumentenkörpers 11 hineingeführt. An das Rohr 17 wird eine Leitung angeschlossen, mittels derer beispielsweise eine 0,9 %-ige Kochsalzlösung zugeführt wird. Das axial eingeführte Rohr 18 ist an einen Druckluftanschluß angelegt, so daß es mit Druckluft als Trägergas beaufschlagt werden kann.

Innerhalb des Hohlraumes 19 des Instrumentenkörpers 11 weisen die Rohre 17, 18 Öffnungen 20, 21 auf, durch welche die Kochsalzlösung bzw. die Luft so in den Innenraum 19 hineingedrückt werden, daß es zu einer Zerstäubung der Kochsalzlösung kommt, d. h. zur Bildung eines Aerosols 20, welches aufgrund des Druckaufbaus insbesondere durch das Rohr 18 in Richtung des Pfeiles durch den Instrumentenkörper 11 zum distalen Ende 13 hin strömt und dort als kegelförmig erweiterndes Aerosolbündel bzw. Aerosolstrahl 22' austritt, welches bzw. welcher somit die Spitze der Schneidelektrode 12 und das von dieser zu koagulierende Biogewebe umgibt. Durch die hohe Spannung der Schneidelektrode 12 wird das Aerosol ionisiert und somit eine ionisierte Strecke zwischen Schneidelektrode 12 und Biogewebe gebildet, auf der sich dann ein besonders gleichmäßiger und damit auch besonders gut lokalisierbarer Lichtbogen ausbildet.

Das Druckluft-Zufuhrrohr 18 ist derart weit axial in das Innere des Instrumentenkörpers 11 hineingeführt, daß seine Austrittsdüse 20 sich unmittelbar oberhalb und etwas hinter der quer verlaufenden Austrittsdüse 21 des Flüssigkeits-Zufuhrrohres 17 befindet. Auf diese Weise saugt die über die Austrittsdüse 21 strömende Luft Flüssigkeit aus dem Rohr 17 an und zerstäubt sie gleichzeitig zur Bildung des Aerosols 22.

Statt der durch die Rohre 17, 18 gebildeten Zerstäubungsvorrichtung könnte auch ein Ultraschallvernebler am bzw. im Instrumentenkörper 11 oder in einem Abstand von diesem vorgesehen sein. Im letzteren Fall wäre der Ultraschallvernebler über einen Aerosol-Zuleitungsschlauch mit dem Inneren des Instrumentenkörpers 11 zu verbinden.

## Patentansprüche

1. Verfahren zur berührungslosen Oberflächenkoagulation von Biogewebe mittels eines Lichtbogens, der durch die von einem Hochfrequenzgenerator erzeugte Hochspannung an einem hochfrequenzchirurgischen Instrument erzeugt wird,
dadurch **gekennzeichnet,**
daß zur Schaffung einer ionisierten Strecke für die erleichterte Lichtbogenbildung ein unter Verwendung einer Flüssigkeit und eines Trägergases gebildeter Aerosolstrahl in die Region, wo der Lichtbogen sich bilden soll und sich eine an einer Hochfrequenzspannung angelegte Elektrode (12) befindet, geschickt wird.

2. Verfahren nach Anspruch 1,
dadurch **gekennzeichnet,**
daß dem Aerosolstrahl zur Verbesserung der Leitfähigkeit Ionen, z. B. NaCl oder KJ, beigemischt werden.

3. Verfahren nach Anspruch 1 oder 2,
dadurch **gekennzeichnet,**
daß der Aerosolstrahl durch eine 0,9 %-ige Kochsalzlösung gebildet wird.

4. Verfahren nach einem der vorhergehenden Ansprüche,
dadurch **gekennzeichnet,**
daß das Aerosol nach dem Zerstäuberprinzip und insbesondere durch einen wie ein Vergaser ausgebildeten Zerstäuber erzeugt wird.

5. Verfahren nach einem der Ansprüche 1 bis 3,
dadurch **gekennzeichnet,**
daß das Aerosol durch einen Ultraschallvernebler erzeugt wird.

6. Verfahren nach einem der vorhergehenden Ansprüche,
dadurch **gekennzeichnet,**
daß als Trägergas für die Bildung des Aerosols, d. h. für die Zerstäubung der im Aerosol enthaltenen Flüssigkeit Luft und/oder Kohlendioxid und/oder Stickstoff verwendet wird, wobei bevorzugt der Vordruck des Trägergases einstellbar ist.

7. Elektrochirurgische Vorrichtung mit einer an eine von einem Hochfrequenzgenerator erzeugte Hochfrequenzspannung anlegbaren Aktivelektrode (12) und einer Neutralelektrode zur Erzeugung eines Lichtbogens im Biogewebe zwecks berührungsmoser Oberflächenkoagulation, insbesondere zur Ausführung des Verfahrens nach einem der Ansprüche 1 bis 6,
dadurch **gekennzeichnet,**
daß sie eine Aerosolstrahl-Bildungkomponente (17,18,19) aufweist, welche einen unter Verwendung eines Trägergases und einer Flüssigkeit gebildeten Aerosolstrahl in den Bereich um die aktive Elektrode (12) schickt, wo der Lichtbogen gebildet werden soll.

8. Vorrichtung nach Anspruch 7,
dadurch **gekennzeichnet,**
daß die Aerosolstrahl-Erzeugungskomponente einen Zerstäuberkanal (19) mit einer Austrittsdüse (13) aufweist und/oder daß die Aerosolstrahl-Erzeugungskomponente einen Ultraschallvernebler umfaßt, der wahlweise im Handgriff (11) oder außerhalb des Handgriffs angeordnet und durch eine Schlauchverbindung mit dem Handgriff (11) verbunden ist und/oder daß die aktive Hochfrequenzelektrode (12) vorzugsweise mittig im Zerstäuber- bzw. Ultraschallnebelkanal (19) angeordnet ist und/oder daß die Zerstäuber-/Vernebler-Elektrodenanordnung in ein und demselben Handgriff (11) untergebracht ist, wobei der bzw. die Hochfrequenzanschlüsse (15) und die Trägergas-Flüssigkeitsanschlüsse (17, 18) ebenfalls am Handgriff (11) vorgesehen sind.

9. Vorrichtung nach Anspruch 7 oder 8,
dadurch **gekennzeichnet,**
daß die aktive Elektrode (12) gegenüber der Austrittsdüse (13) des Instrumentenkörpers (11) etwas zurückversetzt ist und/oder daß die aktive Elektrode (12) nadel- oder stabartig ausgebildet ist und/oder daß der distale Bereich des Instrumentenkörpers (11) aus Isoliermaterial (11') besteht.

10. Vorrichtung nach einem der Ansprüche 7 bis 9,
dadurch **gekennzeichnet,**
daß die aktive Elektrode (12) an eine sich im Instrumentenkörper (11) längs erstreckende Hochfrequenz-Zuleitung (12') angeschlossen ist, die von einer Isolierung (14) umgeben ist und/oder daß zwischen der Zuleitung (12') zur aktiven Elektrode (12) bzw. der Isolation (14) der Zuleitung (12') und der Innenwand des Instrumentenkörpers (11) vorzugsweise isolierende Abstandshalter (16) derart angeordnet sind, daß zwischen an einer bestimmten axialen Stelle des Instrumentenkörpers (11) angeordneten Abstandshaltern (16) ausreichend Platz für den axialen Durchgang des Aerosols vorhanden ist.
